# EUROPEAN PATENT SPECIFICATION

(11) **EP 4 410 996 B1**
(45) Date of publication and mention of the grant of the patent: **01.04.2026**
(21) Application number: 22876985.7
(22) Date of filing: 30.09.2022
(51) Int. Cl.: C12Q 1/6883

(54) **EXOSOME-DERIVED MICRORNA BIOMARKER FOR DIAGNOSIS OF PREMATURE OVARIAN INSUFFICIENCY (POI) AND USE THEREOF**
EXOSOM-ABGELEITETER MIKRORNA-BIOMARKER ZUR DIAGNOSE VON VORZEITIGER OVARIALINSUFFIZIENZ (POI) UND VERWENDUNG DAVON
BIOMARQUEUR DE MICROARN DÉRIVÉ D'EXOSOMES POUR LE DIAGNOSTIC DE L'INSUFFISANCE OVARIENNE PRÉCOCE (POI) ET SON UTILISATION

(30) Priority: 01.10.2021 KR 20210130559; 29.09.2022 KR 20220124256
(43) Date of publication of application: 07.08.2024
(73) Proprietor: Gil Medical Center, Incheon 21565 (KR)
(72) Inventor: YOON, Mee-Sup, Seongnam-si, Gyeonggi-do 13120 (KR); CHON, Seung-Joo, Seongnam-si, Gyeonggi-do 13120 (KR); UMAIR, Zobia, Seongnam-si, Gyeonggi-do 13120 (KR)
(74) Representative: Office Freylinger
(86) International application number: PCT/KR2022/014852
(87) International publication number: WO 2023/055214

(56) References cited:
- WO-A1-2019/022605
- CN-A- 106 520 915
- CN-A- 106 755 544
- KR-A- 20200 025 961
- KR-A- 20210 110 073
- US-A1- 2013 143 764
- US-A1- 2014 378 335
- GUO YING ET AL: "Role of microRNAs in premature ovarian insufficiency", REPRODUCTIVE BIOLOGY AND ENDOCRINOLOGY, vol. 15, no. 1, 12 May 2017 (2017-05-12), XP093054382, Retrieved from the Internet <URL:http://link.springer.com/content/pdf/10.1186/s12958-017-0256-3.pdf> DOI: 10.1186/s12958-017-0256-3
- X. YANG ET AL: "Differentially expressed plasma microRNAs in premature ovarian failure patients and the potential regulatory function of mir-23a in granulosa cell apoptosis", REPRODUCTION, vol. 144, no. 2, 31 May 2012 (2012-05-31), pages 235 - 244, XP055200220, ISSN: 1470-1626, DOI: 10.1530/REP-11-0371
- DANG YUJIE ET AL: "MicroRNA-379-5p is associated with biochemical premature ovarian insufficiency through PARP1 and XRCC6", CELL DEATH & DISEASE, vol. 9, no. 2, 24 January 2018 (2018-01-24), GB, XP093228291, ISSN: 2041-4889, Retrieved from the Internet <URL:https://www.nature.com/articles/s41419-017-0163-8.pdf> DOI: 10.1038/s41419-017-0163-8
- UMAIR ZOBIA ET AL: "MicroRNA-4516 in Urinary Exosomes as a Biomarker of Premature Ovarian Insufficiency", CELLS, vol. 11, no. 18, 7 September 2022 (2022-09-07), pages 2797, XP093054391, DOI: 10.3390/cells11182797
- WOO EUN YOUNG ET AL: "RE07: Identification of microRNA from urinary exosomes as a Premature ovarian insufficiency biomarker", 1 October 2021 (2021-10-01), pages 487, XP009545169, Retrieved from the Internet <URL:https://www.ksog.org/journal/search.php#>
- ZHANG RUNJU ET AL: "Emerging roles for noncoding RNAs in female sex steroids and reproductive disease", MOLECULAR AND CELLULAR ENDOCRINOLOGY, ELSEVIER IRELAND LTD, IE, vol. 518, 12 July 2020 (2020-07-12), XP086326129, ISSN: 0303-7207, [retrieved on 20200712], DOI: 10.1016/J.MCE.2020.110875
- CHON SEUNG JOO, UMAIR ZOBIA, YOON MEE-SUP: "Premature Ovarian Insufficiency: Past, Present, and Future", FRONTIERS IN CELL AND DEVELOPMENTAL BIOLOGY, vol. 9, XP093054377, DOI: 10.3389/fcell.2021.672890
- QIUWAN ZHANG, SUN JUNYAN, HUANG YATING, BU SHIXIA, GUO YING, GU TINGTING, LI BONING, WANG CHUNHUI, LAI DONGMEI: "Human Amniotic Epithelial Cell-Derived Exosomes Restore Ovarian Function by Transferring MicroRNAs against Apoptosis", MOLECULAR THERAPY-NUCLEIC ACIDS, vol. 16, 7 June 2019 (2019-06-07), US , pages 407 - 418, XP055720170, ISSN: 2162-2531, DOI: 10.1016/j.omtn.2019.03.008
- GUO YING, SUN JUNYAN, LAI DONGMEI: "Role of microRNAs in premature ovarian insufficiency", REPRODUCTIVE BIOLOGY AND ENDOCRINOLOGY, vol. 15, no. 1, 1 December 2017 (2017-12-01), XP093054382, DOI: 10.1186/s12958-017-0256-3
- YOUNG, W. E. ET AL.: "RE07: Identification of microRNA from urinary exosomes as a Premature ovarian insufficiency biomarker", THE 107TH ANNUAL CONGRESS OF KOREAN SOCIETY OF OBSTETRICS AND GYNECOLOGY, 1 October 2021 (2021-10-01) - 2 October 2021 (2021-10-02), pages 487, XP009545169
- UMAIR ZOBIA, BAEK MI-OCK, SONG JISUE, AN SEONA, CHON SEUNG JOO, YOON MEE-SUP: "MicroRNA-4516 in Urinary Exosomes as a Biomarker of Premature Ovarian Insufficiency", CELLS, vol. 11, no. 18, pages 2797, XP093054391, DOI: 10.3390/cells11182797

## Description

### BACKGROUND OD THE INVENTION

### 1. Field of the Invention

The present invention relates to an exosome-derived microRNA biomarker for diagnosis of premature ovarian insufficiency (POI) and a use thereof.

### 2. Description of the Related Art

The ovary is a female reproductive organ that matures eggs and secretes sex hormones such as estrogen, progesterone, and testosterone. It maintains the health of the female reproduction system, and maintains the balance of the hormone production system that plays an important role in maintaining a woman's quality of life. Ovarian function can be confirmed by measuring changes in the level of follicular stimulating hormone (FSH), estradiol, or anti-mullerian hormone (AMH), and decreased ovarian function can lead to hormonal imbalance, premature ovarian failure, polycystic ovarian syndrome, and infertility.

Among these, premature ovarian insufficiency (POI) is a pathological condition in which ovarian function is exhausted before the age of 40, and is manifested through symptoms of amenorrhea or oligomenorrhea, decreased estrogen, and elevated serum follicular stimulating hormone (FSH) concentration. More specifically, if there has been no menstruation for more than 6 months before the age of 40 and the blood level of follicular stimulating hormone (FSH) measured twice at intervals of 1 month or more is confirmed to be more than 40 mIU/mL, premature ovarian insufficiency is diagnosed.

The prevalence of premature ovarian insufficiency is approximately 1%, and patients with premature ovarian insufficiency generally exhibit symptoms seen in menopausal women. Premature ovarian insufficiency increases the likelihood of facial flushing, sweating, sleep disorders, and chronic urogenital atrophy, osteoporosis, coronary artery disease, and Alzheimer's disease due to estrogen deficiency. These accompanying symptoms reduce the patient's quality of life and the survival rate decreases due to the increase in the incidence of other diseases and complications.

It is known that the causes of premature ovarian insufficiency are mainly due to exogenous factors, karyotypic abnormalities, and genetic factors, but in most cases, the cause is unknown (Shelling An, 2010). Some cases of premature ovarian insufficiency may be caused by autoimmune diseases or genetic disorders such as Fragile-X syndrome and Turner syndrome. To determine the cause of premature ovarian insufficiency, much research has been conducted on mutations in various genes that affect the ovarian maturation process, but the exact mechanism of premature ovarian insufficiency has not yet been revealed. As such, the onset of premature ovarian insufficiency is a very heterogeneous and unknown phenomenon. In actual clinical trials, the cause of premature ovarian insufficiency is unknown in approximately 90% of cases.

Once diagnosed with premature ovarian insufficiency, the ovaries rarely respond to attempts to overstimulate them, and the chances of having a live birth are very low. Therefore, it is important to have a test method that can pre-screen prospective patients who will be diagnosed with premature ovarian insufficiency, but the characteristic clinical symptoms that precede premature ovarian insufficiency have not been specifically reported. Thus, it is difficult to predict the likelihood of developing premature ovarian insufficiency unless there is a specific past history (chemotherapy and radiotherapy, ovarian cyst removal, etc.).

Accordingly, in order to diagnose early and find the etiology of premature ovarian insufficiency, various tests such as chromosome testing, glazed X syndrome carrier testing, thyroid disease, pituitary disease, autoimmune disease, previous diagnosis and surgery are being performed. However, there is very little information on the treatment and prognosis of patients for each cause, and none of them are used as screening tests for early diagnosis. In addition, most of the current treatment for patients with premature ovarian insufficiency is treatment and management after diagnosis. In patients with premature ovarian insufficiency, pregnancy attempts have been made through *in vitro* activation (IVA), in which whole ovaries or ovarian tissue/follicles are harvested and reimplanted after *in vitro* culture, but this method is known to have poor results in patients whose ovaries are already irreparably damaged. Therefore, in reality, there are few tests that can predict abnormal declines in ovarian function in advance and recommend prior preparation.

It is advisable to determine whether the probability of developing premature ovarian insufficiency is high and to freeze eggs or attempt pregnancy before premature ovarian insufficiency occurs. However, there is currently no clear marker or test method to detect premature ovarian insufficiency at an early stage, making early diagnosis very difficult.

Meanwhile, Turner syndrome refers to the loss or partial deletion of one of a woman's two X chromosomes, or a structural defect in the X chromosome, that results in ovarian dysfunction. The karyotype of 50% of Turner syndrome is 45 X, lacking one of the two X chromosomes, and the remainder has a karyotype of mosaicism or partial deletion. Therefore, karyotyping is used to analyze Turner syndrome.

Turner syndrome is known to be one of the genetic causes of premature ovarian insufficiency. Therefore, it is necessary to discover a diagnostic marker that can distinguish between premature ovarian insufficiency due to Turner syndrome and premature ovarian insufficiency due to other causes.

Exosomes are microscopic vesicles containing substances that carry cellular RNA and proteins. Exosomes are membrane-structured vesicles secreted from various types of cells, and are known to play a variety of roles, including binding to other cells and tissues and delivering membrane components, proteins, and RNA. Originally, exosomes were discovered during the final stage of red blood cell maturation, when intracellular proteins are released and removed, leaving only hemoglobin within the red blood cells. In addition, in a study using an electron microscope, it was observed that exosomes do not directly break away from the plasma membrane, but rather originate from specific intracellular compartments called multivesicular bodies (MVBs) and are released and secreted out of the cell. When the multivesicular bodies and the plasma membrane fuse, vesicles are released into the extracellular environment, called exosomes.

Recently, exosomes have received much attention as markers for disease diagnosis and as intercellular signaling carriers. The exosome separation and analysis method is one of the liquid biopsy methods selected as one of the top 10 promising future technologies by MIT in 2015. Liquid biopsy is recognized as a next-generation treatment strategy because it allows detailed observation of the occurrence and spread of disease by simply examining blood or body fluids in a non-invasive way. In addition, exosomes are known to be secreted from various cells and circulate through plasma, cerebrospinal fluid, and urine. Exosomes are small, stable endoplasmic reticulum (30-200 nm) structures wrapped in a phospholipid bilayer and contain the same genetic and protein information as the parent cell. In particular, exosomes contain microRNA (miRNA), which can be used as useful markers for molecular diagnosis such as early diagnosis of cancer. Exosomes also play an important role in signaling between cells. Accordingly, exosomes can act as carriers of factors that cause premature ovarian insufficiency and can enter not only granulosa cells but also other surrounding cells, such as endometrial stromal cells, to regulate the growth and differentiation process of the surrounding cells.

In biology, the role of miRNAs is an area of intense interest as they have already been extensively studied in determining altered protein function in various diseases such as cancer. MiRNAs are present in the mammalian ovary and their expression patterns are altered throughout ovarian development and follicle formation, and are expected to play a functional role in the ovarian cycle. Although miRNAs have been reported to play a role in diseases such as cancer and neurological disorders as regulators that suppress gene expression, the profile of premature ovarian insufficiency-related miRNAs isolated from premature ovarian insufficiency patients and their functional roles in premature ovarian insufficiency have not been identified.

Existing inventions of diagnostic methods for premature ovarian insufficiency include a method for diagnosing through VEGF (vascular endothelial growth factor), which is a neovascularization gene (Korean Patent Registration No. 10-01301968), and a method for diagnosing using a microarray with DNA specific to premature menopause patients (Korean Patent Registration No. 10-0861493). However, all of the above methods have the disadvantage that the target is DNA or blood, which makes it difficult or impossible to diagnose early.

Accordingly, the present inventors confirmed miRNA expression profiles in exosomes isolated from urine of premature ovarian insufficiency patients, and identified miRNAs associated with premature ovarian insufficiency caused by Turner syndrome and premature ovarian insufficiency caused by factors other than Turner syndrome, and screened and validated miRNAs that can be utilized as diagnostic markers for premature ovarian insufficiency. In addition, the present inventors completed the present invention by confirming the expression of miRNA in a mouse model of premature ovarian insufficiency and by performing protein expression analysis to determine the mechanism of action of the miRNA in premature ovarian insufficiency.

### SUMMARY OF THE INVENTION

The invention is defined in the appended claims.

It is an object of the present invention tc provide a method for diagnosing premature ovarian insufficiency.

It is another object of the present invention to provide a method for screening a therapeutic agent for premature ovarian insufficiency.

To achieve the above objects, the present document describes a composition for diagnosing premature ovarian insufficiency, which includes an agent capable of measuring the expression level of one or more miRNAs selected from the group consisting of miR-4516, miR-29a-3p, and miR-30b-5p.

Also disclosed but not part of the invention is a kit for diagnosing premature ovarian insufficiency comprising the said diagnostic composition.

Also disclosed is a method for diagnosing premature ovarian insufficiency comprising the following steps:
1) a step of collecting a biological sample from an individual, separating exosomes from the sample, and isolating RNA from the exosomes; and
2) a step of measuring the expression level of miRNAs consisting of miR-4516, miR-29a-3p, and miR-30b-5p in the isolated RNA, and comparing the expression level with the expression level of the gene in the normal control group.

Also disclosed is a method for screening a therapeutic agent for premature ovarian insufficiency comprising the following steps:
1) a step of treating a test substance to cells having a nucleotide sequence encoding miRNAs consisting of miR-4516, miR-29a-3p, and miR-30b-5p *in vitro*; and
2) a step of analyzing the miRNA expression in the isolated cells treated with the test substance and comparing the expression pattern of the miRNA with the expression pattern of the miRNA in the isolated control cells not treated with the test substance.

### ADVANTAGEOUS EFFECT

By using the biomarker of the present invention, early diagnosis of premature ovarian insufficiency is possible, diagnosis differentiating between premature ovarian insufficiency and premature ovarian insufficiency caused by Turner syndrome is possible, and a method for screening a therapeutic agent for premature ovarian insufficiency through a change in the expression of the biomarker can be provided.

### BRIEF DESCRIPTION OF THE DRAWINGS

Figure 1 is a set of photographs showing the exosomes isolated from urine collected from a patient with premature ovarian insufficiency and observed with an electron microscope.
Figure 2 is a set of photographs showing the exosome markers CD9, CD63, and TSG101 in the exosomes isolated from urine.
Figure 3 is a graph showing the size of the exosomes isolated from urine.
Figure 4 is a heatmap showing the changes in the expression of miRNA genes in a patient group with premature ovarian insufficiency and a normal control group.
Figure 5 is a graph showing the number of target genes of 33 miRNAs that are differentially expressed in patients with premature ovarian insufficiency, counted by the category to which they belong.
Figure 6 is a graph showing the results of confirming the expression of 12 selected miRNAs that showed differences in the expression between the premature ovarian insufficiency patient group without Turner syndrome, and the normal control patient group among the premature ovarian insufficiency patient group, using the results of miRNA sequencing and qRT-PCR.
Figure 7 is a graph showing the results of confirming the expression of 12 selected miRNAs that showed differences in the expression between the premature ovarian insufficiency patient group with Turner syndrome, and the normal control patient group among the premature ovarian insufficiency patient group, using the results of miRNA sequencing and qRT-PCR.
Figure 8 is a graph showing the expression level of miR-4516 for each group (control: normal control group, Turner: premature ovarian insufficiency patient group with Turner syndrome, POI: premature ovarian insufficiency patient group without Turner syndrome).
Figure 9 is a graph showing the expression level of miR-29a-3p for each group (control: normal control group, Turner: premature ovarian insufficiency patient group with Turner syndrome, POI: premature ovarian insufficiency patient group without Turner syndrome).
Figure 10 is a graph showing the expression level of miR-30b-5p for each group (control: normal control group, Turner: premature ovarian insufficiency patient group with Turner syndrome, POI: premature ovarian insufficiency patient group without Turner syndrome).
Figure 11a is a set of photographs showing the ovaries of a premature ovarian insufficiency model mouse (bottom) injected with cyclophosphamide (CTX) and busulfan (BUS) and a control group mouse (top).
Figure 11b is a graph comparing the sizes of the ovaries of Figure 11a.
Figure 12a is a set of photographs showing the ovarian sections of a premature ovarian insufficiency model mouse injected with cyclophosphamide (CTX) and busulfan (BUS) and a control group mouse stained with hematoxylin and eosin.
Figure 12b is a graph comparing the numbers of follicles of Figure 12a.
Figure 12c is a graph comparing the ratio of atretic follicles to all follicles of Figure 12a.
Figure 13 is a set of photographs showing the ovarian sections of a premature ovarian insufficiency model mouse injected with cyclophosphamide (CTX) and busulfan (BUS) and a control group mouse stained with Sirius-red.
Figure 14a is a set of photographs showing the ovarian sections of a premature ovarian insufficiency model mouse injected with cyclophosphamide (CTX) and busulfan (BUS) and a control group mouse stained with TUNEL.
Figure 14b is a graph comparing the percentage of TUNEL positive (+) cells to total cells in Figure 14a.
Figure 15 is a set of photographs showing the ovarian sections of a premature ovarian insufficiency model mouse injected with cyclophosphamide (CTX) and busulfan (BUS) and a control group mouse stained with Ki67.
Figure 16a is a graph comparing the expression of miR-4516 in the ovaries of a premature ovarian insufficiency model mouse injected with cyclophosphamide (CTX) and busulfan (BUS) and a control group mouse.
Figure 16b is a graph comparing the expression of miR-4516 in the uterus of a premature ovarian insufficiency model mouse injected with cyclophosphamide (CTX) and busulfan (BUS) and a control group mouse.
Figure 17 is a diagram showing the binding site in the 3'-UTR of STAT3, the target gene of miR-4516 and miR-4516.
Figure 18a is a diagram showing the expression levels of STAT3, BAX, and p53 in the ovaries of a premature ovarian insufficiency model mouse injected with cyclophosphamide (CTX) and busulfan (BUS) and a control group mouse.
Figure 18b is a graph comparing the expression level of STAT3 of Figure 18a.
Figure 18c is a graph comparing the expression level of BAX of Figure 18a.
Figure 18d is a graph comparing the expression level of p53 of Figure 18a.
Figure 19 is a diagram showing the mechanism of action of miR-4516 in the ovary.

### DESCRIPTION OF THE PREFERRED EMBODIMENTS

Hereinafter, the present invention is described in detail.

The present document describes a composition for diagnosing premature ovarian insufficiency and a composition for diagnosing premature ovarian insufficiency due to Turner syndrome, which includes an agent capable of measuring the expression level of miRNAs consisting of miR-4516, miR-29a-3p, and miR-30b-5p.

If the expression of miR-4516 is increased, it can be diagnosed as premature ovarian insufficiency. If the expression of miR-29a-3p is decreased, it can be diagnosed as premature ovarian insufficiency not caused by Turner syndrome, and if the expression of miR-30b-5p is decreased, it can also be diagnosed as premature ovarian insufficiency not caused by Turner syndrome. Meanwhile, if the expression of miR-29a-3p is increased, it can be diagnosed as premature ovarian insufficiency caused by Turner syndrome, and if the expression of miR-30b-5p is increased, it can also be diagnosed as premature ovarian insufficiency caused by Turner syndrome.

If the expression of miR-4516 is increased and the expression of miR-29a-3p is decreased, it can be diagnosed as premature ovarian insufficiency not caused by Turner syndrome, and if the expression of miR-4516 is increased and the expression of miR-30b-5p is decreased, it can also be diagnosed as premature ovarian insufficiency not caused by Turner syndrome. On the other hand, if the expressions of miR-4516 and miR-29a-3p are increased or if the expressions of miR-4516 and miR-30b-5p are increased, it can be diagnosed as premature ovarian insufficiency caused by Turner syndrome.

If the expression of miR-4516 is increased and the expressions of miR-29a-3p and miR-30b-5p are decreased, it can be diagnosed as premature ovarian insufficiency not caused by Turner syndrome. Meanwhile, if the expressions of miR-4516, miR-29a-3p, and miR-30b-5p are all increased, it can be diagnosed as premature ovarian insufficiency caused by Turner syndrome.

The said agent may be a nucleic acid containing a polynucleotide identical to or complementary to the nucleic acid sequence of the miRNA.

The complementary polynucleotide means that the antisense oligonucleotide is sufficiently complementary to selectively hybridize to the miRNA target under predetermined hybridization or annealing conditions, preferably physiological conditions, and includes both substantially complementary and perfectly complementary polynucleotides. Substantially complementary means not completely complementary, but complementary enough to produce a sufficient effect by binding to the target sequence.

The nucleic acid may be a primer or probe. Specifically, it includes polynucleotides, oligonucleotides, DNA, RNA, and analogs thereof and derivatives thereof, for example, peptide nucleic acids (PNA) or mixtures thereof. In addition, the nucleic acid may be single or double stranded and can encode molecules including polypeptides, mRNA, microRNA, or siRNA.

The primer or probe may be labeled with a fluorescent substance, a chemiluminescent substance, or a radioactive isotope at its end or inside thereof.

The miRNA may be RNA obtained from exosomes isolated from a biological sample.

The biological sample may be cell culture supernatant, whole blood, serum, plasma, ascitic fluid, cerebrospinal fluid, bone marrow aspirate, bronchoalveolar lavage, urine, vaginal fluid, mucus, saliva, sputum, or a lysate purified from a biosample. Preferably, the biological sample may be urine or a lysate purified from a biosample.

The present document also describes a kit for diagnosing premature ovarian insufficiency comprising the said diagnostic composition. In addition, the present invention provides a kit for diagnosing premature ovarian insufficiency caused by Turner syndrome comprising the said diagnostic composition.

The kit may further comprise samples necessary for the diagnosis of premature ovarian insufficiency. The kit may include polymerase, buffer, nucleic acid, coenzyme, fluorescent substance, or a combination thereof for the detection of nucleic acid. The polymerase may preferably be Taq polymerase.

Also disclosed is a method for diagnosing premature ovarian insufficiency comprising the following steps:
1) a step of collecting a biological sample from an individual, separating exosomes from the sample, and isolating RNA from the exosomes; and
2) a step of measuring the expression level of miRNAs consisting of miR-4516, miR-29a-3p, and miR-30b-5p in the isolated RNA, and comparing the expression level with the expression level of the gene in the normal control group.

If the expression level of miR-4516 is increased compared to the normal control group, or the expression level of miR-29a-3p or miR-30b-5p is decreased compared to the normal control group, the individual can be determined to be at high risk of premature ovarian insufficiency. In addition, if the expression level of miR-29a-3p or miR-30b-5p is increased compared to the normal control group, the subject can be determined to have premature ovarian insufficiency caused by Turner syndrome.

The individual may be a mammal, and may be a human, dog, cat, rat, mouse, hamster, rabbit, horse, sheep, cow, goat or pig. The individual may preferably be a human.

The term "diagnosis" refers to determining a disease, and may include the name of the disease, condition, stage, etiology, and the like of premature ovarian insufficiency.

The exosomes may be in a spherical shape with an average diameter of 50 to 60 nm, preferably in a spherical shape with an average diameter of about 57 nm.

The expression level of the miRNA can be measured by electrophoresis, microarray, Northern blotting, PCR (polymerase chain reaction), reverse transcription-PCR (RT-PCT), real-time PCR (qRT-PCR), or a combination thereof.

In addition, also disclosed is a method for screening a therapeutic agent for premature ovarian insufficiency comprising the following steps:
1) a step of treating a test substance to cells having a nucleotide sequence encoding miRNAs consisting of miR-4516, miR-29a-3p, and miR-30b-5p *in vitro*; and
2) a step of analyzing the miRNA expression in the isolated cells treated with the test substance and comparing the expression pattern of the miRNA with the expression pattern of the miRNA in the isolated control cells not treated with the test substance.

If the expression level of miR-4516 in cells treated with the test substance is decreased compared to the expression level in the control cells not treated with the test substance; or if the expression level of miR-29a-3p or miR-30b-5p in cells treated with the test substance is increased compared to the expression level in the control cells not treated with the test substance, the test substance can be a candidate for the treatment of premature ovarian insufficiency.

In addition, the expression level of miR-4516 in cells treated with the test substance is decreased compared to the expression level of control cells not treated with the test substance; or the expression level of miR-29a-3p or miR-30b-5p is decreased in cells treated with the test substance is decreased compared to the expression level of control cells not treated with the test substance, the test substance can be a candidate for the treatment of premature ovarian insufficiency caused by Turner syndrome.

Throughout the specification, "premature ovarian insufficiency" refers to both premature ovarian insufficiency caused by Turner syndrome and premature ovarian insufficiency not caused by Turner syndrome. And "premature ovarian insufficiency patient group" refers to both the premature ovarian insufficiency patient group caused by Turner syndrome and the premature ovarian insufficiency patient group not caused by Turner syndrome.

The "premature ovarian insufficiency caused by Turner syndrome" refers specifically to premature ovarian insufficiency due to Turner syndrome among premature ovarian insufficiency, and the "premature ovarian insufficiency patient group caused by Turner syndrome" refers specifically to the group of patients with premature ovarian insufficiency caused by Turner syndrome.

Patients with premature ovarian insufficiency are those who have had no menstruation for more than 6 months before the age of 40 and have chronic rare ovulation or anovulation with serum folliclestimulating hormone (FSH) of more than 40 mIU/mL measured twice at intervals of more than 1 month.

Specifically, it can be confirmed by measuring changes in follicular stimulating hormone (FSH) and estradiol levels.

Turner syndrome refers to a chromosomal condition that affects female development. Turner syndrome is a condition in which one normal X chromosome is present and the other sex chromosome is absent or structurally altered, including the case of mosaicism of chromosomes with the karyotype (45,X), which is the karyotype of Turner syndrome. Turner syndrome is characterized by early loss of ovarian function and may present with short stature, impaired sexual development, heart disease, otitis media, venous vessels, lymphedema, kidney malformations, hypothyroidism, obesity, diabetes, hypertension, osteoporosis, and chronic colitis.

Some cases of premature ovarian insufficiency are caused by Turner syndrome. Although women with Turner syndrome do not necessarily exhibit premature ovarian insufficiency, the risk of premature ovarian insufficiency appears to be high. Therefore, it is necessary to distinguish whether the cause of premature ovarian insufficiency is due to Turner syndrome.

The "miR" or "micro RNA" of the present invention refers to 21 to 23 non-coding RNAs that posttranscriptionally regulate gene expression by promoting degradation of target RNAs or inhibiting their translation. The mature sequence of the miRNA used in this specification can be obtained from the miRNA database (http://www.mirbase.org). In general, micro RNA is transcribed into a precursor about 70-80 nt (nucleotide) long with a hairpin structure called pre-miRNA, and then cleaved by Dicer, an RNAse III enzyme, to produce a mature form. Micro RNA forms a ribonucleocomplex called miRNP, which cleaves target genes or inhibits translation through complementary binding to target sites. More than 30% of human miRNAs exist in clusters, and after being transcribed as a single precursor, the final mature miRNA is formed through a cleavage process.

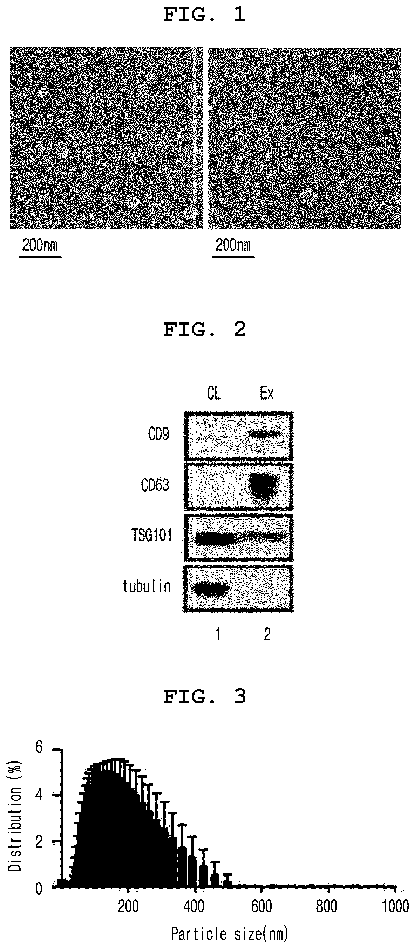

In a specific experimental example of the present invention, patients with premature ovarian insufficiency were divided into three groups: a group of patients with premature ovarian insufficiency not caused by Turner syndrome, a group of patients with premature ovarian insufficiency caused by Turner syndrome, and a normal control group. Urine was collected from the experimental and control groups, and exosomes were isolated. The isolated exosomes were confirmed to have a spherical shape with an average diameter of 57.022 nm (Figure 1), and the exosomes could be identified by confirming the expression of the exosome markers CD9, CD63, and TSG101 (Figure 2). In addition, it was confirmed that the isolated exosomes exhibited a typical exosome distribution pattern and that the size of the exosomes was about 50 to 300 nm (Figure 3).

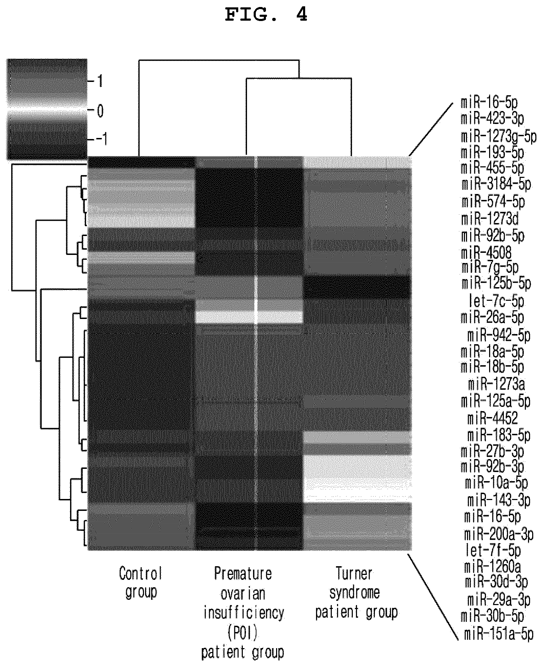

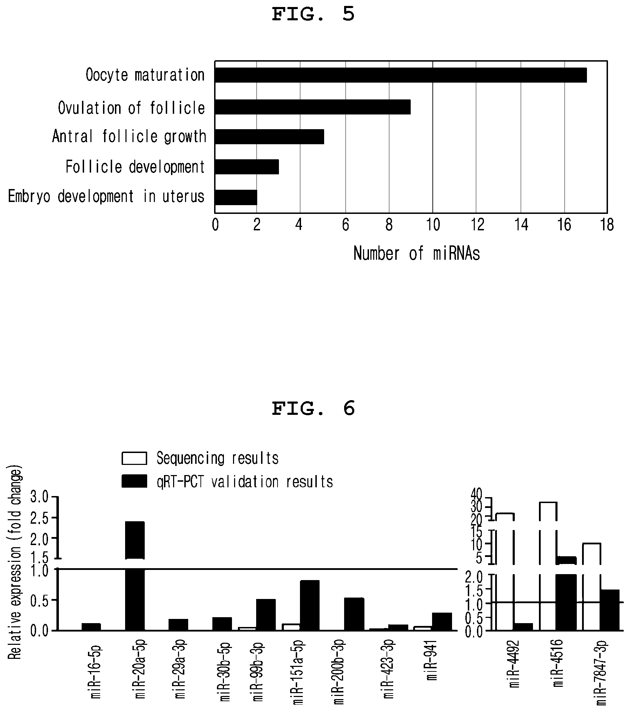

Libraries were formed with RNAs from the normal control group and the groups of patients with premature ovarian insufficiency (including both groups of patients with premature ovarian insufficiency caused by Turner syndrome and not caused by Turner syndrome), and genes with increased or decreased expression in the normal control and premature ovarian insufficiency patients groups (including both groups of patients with premature ovarian insufficiency caused by Turner syndrome and not caused by Turner syndrome) were confirmed (Figure 4). By confirming the biological functions of the above genes, it was confirmed that miRNAs with differences in expression are associated with egg growth and maturation (Figure 5).

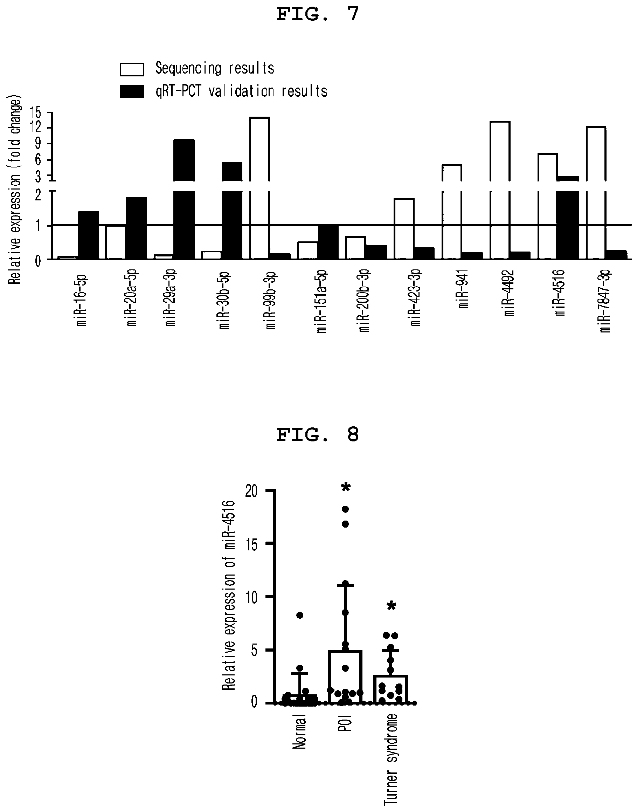

Among the above miRNAs, 12 miRNAs were selected and their expressions were confirmed in the normal control group and the group of patients with premature ovarian insufficiency not caused by Turner syndrome. (Figures 6 and 7). Among the above miRNAs, the miRNAs that showed differences similar to those in sequencing between the normal control group and the group of patients with premature ovarian insufficiency not caused by Turner syndrome were miR-16-5p, miR-20a-5p, miR-29a-3p, miR-30b-5p, miR-99b-3p, miR-151a-5p, miR-200b-3p, miR-423-3p, miR-941, miR-4516, miR-4492, and miR-7847-3p. In addition, the expressions of 12 miRNAs were confirmed in a validation cohort of the group of patients with premature ovarian insufficiency caused by Turner syndrome and the normal control group (Figure 6). Among the above miRNAs, the miRNAs that showed differences similar to those in sequencing between the normal control group and the group of patients with premature ovarian insufficiency caused by Turner syndrome were miR-20a-5p and miR-200b-3p (Figure 7).

When the expression levels of the above miRNAs were examined in each patient group, miR-4516 was expressed significantly higher in all the groups of patients with premature ovarian insufficiency than in the normal control group (Figure 8).

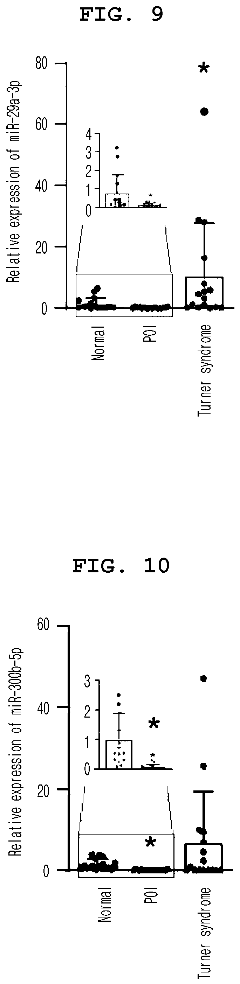

The expression levels of miR-29a-3p and miR-30b-5p were increased in the group of patients with premature ovarian insufficiency caused by Turner syndrome compared to the normal control group, but decreased in the group of patients with premature ovarian insufficiency not caused by Turner syndrome (Figures 9 and 10).

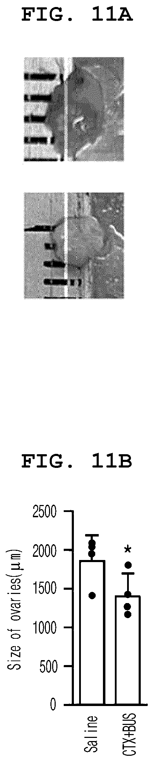

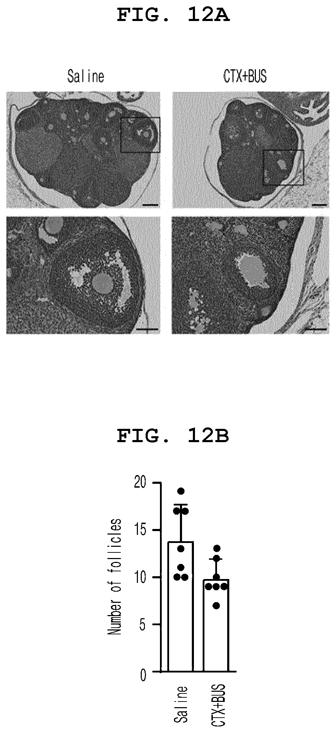

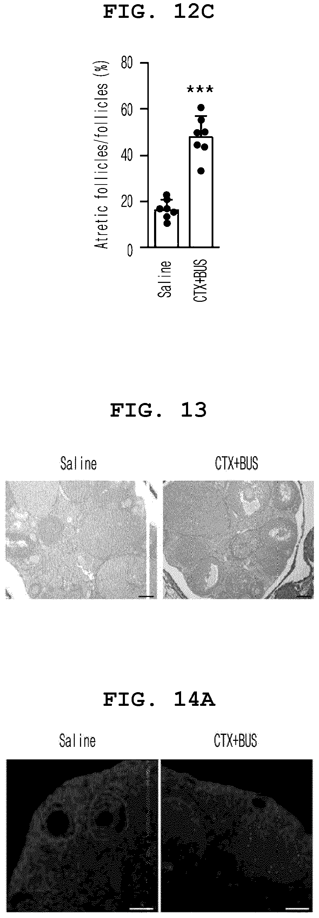

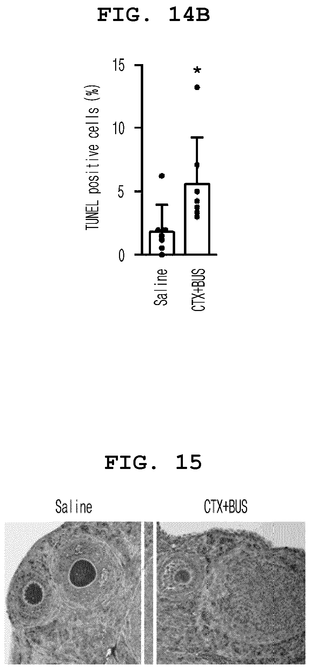

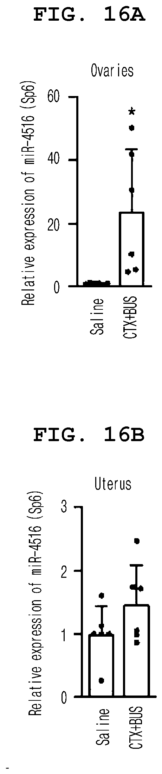

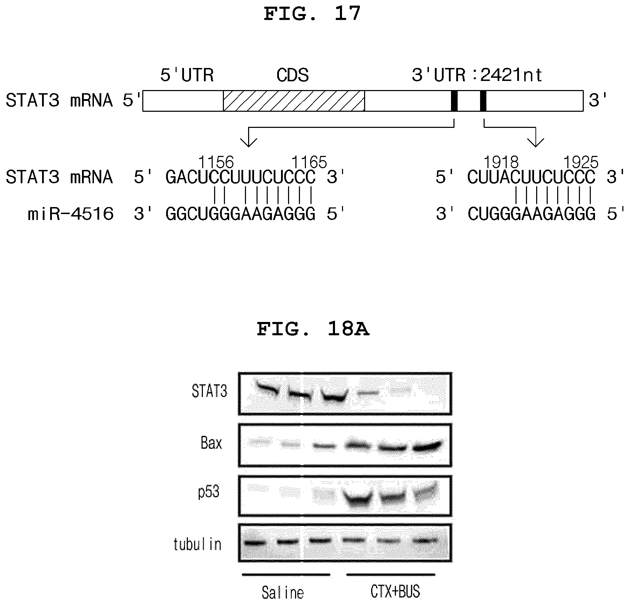

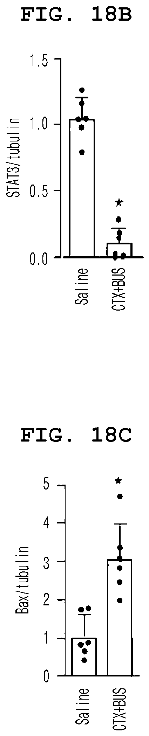

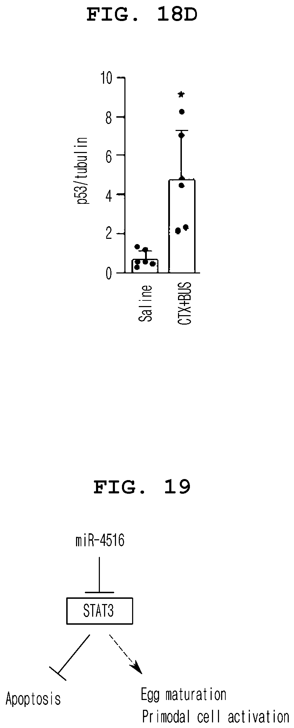

In a mouse model of premature ovarian insufficiency injected with CTX and BUS, the ovary size was decreased (Figures 11a and 11b), the number of follicles was decreased (Figures 12a and 12b), and the proportion of atretic follicles was increased (Figure 12c). In addition, the reactivity of Sirius-red staining, which indicates collagen accumulation, was increased, indicating that tissue fibrosis was progressing (Figure 13). Additionally, as a result of TUNEL analysis, apoptosis was increased (Figures 14a and 14b), and as a result of Ki67 staining, the number of cells stained with Ki67 was decreased (Figure 15). In addition, it was confirmed that the expression of miR-4516 was increased in the ovaries of the premature ovarian insufficiency mouse model, but not in the uterus (Figures 16a and 16b). Through analysis of miRTarBase2020, STAT3 was selected as the target gene of miR-4516, and two binding sites of miR-4516 were identified in STAT3 3'-UTR (Figure 17). It was confirmed that the expression of STAT3 was decreased in the ovaries of the premature ovarian insufficiency mouse model (Figures 18a to 18d). Based on the above results, it is assumed that miR-4516 promotes egg cell death, follicle generation, and primodal cell activity through regulating the expression of STAT3 (Figure 19).

Hereinafter, the present invention will be described in detail by the following experimental examples.

However, the following experimental examples are only for illustrating the present invention, and the contents of the present invention are not limited thereto. It is well understood by those in the art who has the average knowledge on this field that the embodiments of the present invention are given to explain the present invention more precisely.

### Experimental Example 1: miRNA sequencing and biomarker screening

### <1-1> Selection of sequencing cohort for mRNA sequencing

Normal people without premature ovarian insufficiency were selected as the control group, and among patients with premature ovarian insufficiency, a group of patients with premature ovarian insufficiency not caused by Turner syndrome and a group of patients with premature ovarian insufficiency caused by Turner syndrome were selected as the experimental groups.

According to the diagnostic method of premature ovarian insufficiency, women with chronic rare ovulation or anovulation with blood folliclestimulating hormone (FSH) of more than 40 mIU/mL measured twice at intervals of more than 1 month were considered as patients with premature ovarian insufficiency, while women with regular menstrual cycles and normal ovulation were selected as the control group.

For each group, 7 patients with premature ovarian insufficiency not caused by Turner syndrome, 7 patients with premature ovarian insufficiency caused by Turner syndrome, and 5 normal people were selected and analyzed for age, height, weight, BMI, etc. (see Tables 1 and 2).

**[Table 1]**

| | Group of patients with premature ovarian insufficie ncy not caused by Turner syndrome (N=7) | Group of patients with premature ovarian insufficie ncy caused by Turner syndrome (N=7) | Normal control group (N=5) | p-value |
|---|---|---|---|---|
| Age (year) | 37.7±8.9 | 31.0±6.1 | 34.8±5.0 | 0.226 |
| Height (cm) | 161.5±6.2 | 148.1±6.2 | 160.9±7.3 | 0.002 |
| Weight (kg) | 56.9±8.3 | 53.3±6.4 | 54.8±5.0 | 0.622 |
| BMI (Body mass index) (kg/m²) | 21.8±2.7 | 24.4±3.6 | 21.2±1.5 | 0.105 |
| SBP (systolic blood pressure) (mmHg) | 12 6. 4±3. 4 | 135.1±7.9 | 123.3±3.8 | 0.003 |
| DBP (diastolic blood pressure) (mmHg) | 75.1±8.4 | 80.9±7.3 | 80.0±1.7 | 0.258 |
| AST (Aspartate aminotransferase test) (IU/L) | 20.0±7.7 | 27.1±16.5 | 15.0±2.9 | 0.162 |
| ALT (Alanine aminotransferase test) (IU/L) | 15.3±6.3 | 37.7±40.1 | 14.7±4.3 | 0.165 |
| Total cholesterol (mg/dL) | 189.0±39.7 | 202.1±31.3 | 162.2±19.0 | 0.101 |
| Triglyceride (mg/dL) | 127.9±25.2 | 222.9±245. 2 | 77.5±22.5 | 0.219 |
| HDL-C (highdensity lipoprotein) (mg/dL) | 73.9±18.2 | 80.3±17.5 | 56.8±14.2 | 0.063 |
| LDL-C (lowdensity lipoprotein) (mg/dL) | 98.1±31.6 | 91.9±9.3 | 96.9±11.7 | 0.839 |
| Prolactin (ng/mL) | 8.5±2.8 | 7.9±2.2 | 9. 1±1. 4 | 0.655 |
| Thyroid stimulating hormone (mIU/mL) | 1.2±0.6 | 2.6±1.1 | 1. 9±0. 4 | 0.010 |
| Follicular stimulating hormone (mIU/mL) | 114.8±35.1 | 52.8±15.5 | 7.1±0.7 | <0.001 |
| Estradiol (pg/mL) | 24.6±16.7 | 15.7±4.5 | 39.6±4.2 | 0.003 |

In the group of patients with premature ovarian insufficiency not caused by Turner syndrome, total cholesterol was slightly higher and triglyceride was approximately 1.9 times higher than in the normal control group. In addition, HDL cholesterol (HDL-C) was somewhat high, prolactin was also somewhat elevated, follicular stimulating hormone (FSH) was very high, and estradiol, on the other hand, was found to be secreted at only about 17% compared to the normal control group. Patients with premature ovarian insufficiency caused by Turner syndrome and tend to be shorter than normal controls, with no significant difference in weight. They also have a high BMI index, which is in the overweight range. This is consistent with the developmental characteristics of Turner syndrome, which is characterized by slow growth, with a final height in the 140 cm range, and a tendency to gain weight because calories are not burned well compared to the amount of food consumed, leading to obesity or diabetes.

Liver indexes, AST and ALT, were also measured higher than those in the normal control group. Total cholesterol levels were high, and HDL cholesterol levels were also high. Triglycerides were higher than in normal controls but lower than in patients with premature ovarian insufficiency not caused by Turner syndrome, and thyroid-stimulating hormones were secreted in the highest amount among the three groups. In the case of Turner syndrome, hypothyroidism frequently occurs, so the level of thyroid stimulating hormone is high.

Prolactin was lower in patients with premature ovarian insufficiency caused by Turner's syndrome than in normal controls, but not in patients with premature ovarian insufficiency not caused by Turner's syndrome. In patients with Turner syndrome, follicular stimulating hormone was secreted more than in the normal controls, but less than in patients with premature ovarian insufficiency, and estradiol was secreted lower than in patients with premature ovarian insufficiency. This is a characteristic of Turner syndrome, which involves insufficient secretion of female hormones, which often causes symptoms such as osteoporosis.

**[Table 2]**

| | Age of menarche (year) | Amenorr hea period (month) | Karyotype | FMR-1 gene variation |
|---|---|---|---|---|
| Group of patients with premature ovarian insufficiency not caused by Turner syndrome | | | | |
| 1 | 15 | 18 | 46,XX | No variation |
| 2 | 13 | 24 | 47,XXX[39]1/46,XX[1] | No variation |
| 3 | 15 | 24 | 46,XX | No variation |
| 4 | 16 | 36 | 46,XX | No variation |
| 5 | 12 | 3 | 46,XX | No variation |
| 6 | 15 | 5 | 46,XX | No variation |
| 7 | 14 | 30 | 46,XX | No variation |

| Group of patients with premature ovarian insufficiency caused by Turner syndrome | | | | |
|---|---|---|---|---|
| 1 | No menarche | | 46,X,i(X)(q10),22pstk+[16] /45,X,22pstk+[14] | No variation |
| 2 | No | | 46,X,i(X)(q10) | No |
| | menarche | | | variation |
| 3 | No menarche | | 45,X | No variation |
| 4 | No menarche | | 46,X,i(X)(q10) | No variation |
| 5 | No menarche | | 46,X,i(X) (q10) [24]/45,X[6] | No variation |
| 6 | No menarche | | 45,X | No variation |
| 7 | No menarche | | 45,X | No variation |

Patients with premature ovarian insufficiency not caused by Turner syndrome started menarche around age 13, had amenorrhea for at least three months, and did not have FMR-1 (Fragile X Messenger Ribonucleoprotein 1) gene mutation, which is the most common single gene mutation associated with premature ovarian insufficiency. Among these, patient 2 had karyotypes of 46,XX and 47,XXX, but as it was not considered to be a direct cause of premature ovarian insufficiency, the patient was classified as a patient with premature ovarian insufficiency not caused by Turner syndrome. Patients with premature ovarian insufficiency caused by Turner syndrome showed primary amenorrhea and did not have a mutation in the FMR-1 gene. Some of these patients had a Turner syndrome karyotype with a typical chromosome number abnormality of 45,X, as in patients 3, 6 and 7, while others had a Turner syndrome karyotypes with a chromosomal structural abnormality such as deletions, as in patients 2 and 4. Patients 1 and 5 showed mosaicism of 46,X,i and 45,X, which is a chromosomal structure abnormality.

### <1-2> Isolation of exosomes

Urine was collected from the experimental and control group subjects selected in Experimental Example 1. The collected urine was first centrifuged at 2000 g for 10 minutes to remove cells and sediments, and then centrifuged at 13,500 g for 20 minutes. Afterwards, it was filtered through a 0.22 µm filter and concentrated again using centrifugation (2000 g, 30 minutes).

The exosome-precipitated solution (Exoquick-TC) was mixed at a ratio of 1:4 and incubated at 4°C overnight. The next day, the incubated solution was centrifuged at 2000 g for 30 minutes to isolate the exosome pellet. The isolated pellet was resuspended, combined with beads for 15 minutes, and then centrifuged at 8000 g for 5 minutes to obtain a supernatant containing exosomes.

### <1-3> Confirmation of exosomes

The supernatant containing exosomes was dissolved using a lysis buffer (Cat. No. 9303, Cell Signaling Technology) and then microcentrifuged at 4°C, 13,000 g for 10 minutes. The supernatant of the centrifuged solution was collected and boiled in sodium dodecyl sulfate (SDS) buffer for 3 minutes, and then the proteins were separated by SDS-PAGE. After electrophoresis, the gel was transferred to a polyvinylidene fluoride membrane (Millipore, Billerica, MA, USA) and the membrane was blocked for 30 minutes in phosphate-buffered saline Tween (PBST) containing 5% (w/v) nonfat skim milk at room temperature and then incubated with a primary antibody in PBST containing 5% (w/v) nonfat skim milk overnight at 4°C. Exosomes were detected using a secondary antibody that binds to horseradish peroxidase using an immobilon Western chemiluminescent HRP substrate (Millipore) .

The detected exosomes were observed with an electron microscopy.

Specifically, it was placed on a copper grid coated with thin carbon foil (Cat. No. 01340, Ted Pella, Inc. USA) and stained with 2% (w/v) uranyl acetate (UrAc) for 1 minute. Then, electron micrographs were taken using a Bio-High voltage EM system (JEM-1400 Plus at 120 kV and JEM-1000BEF at 1000 kV; JEOL Ltd., Japan).

As a result, spherical exosomes with an average diameter of 57.022 nm were observed (Figure 1), and the expressions of CD9, CD63, and TSG101 (Tumor Susceptibility 101), known as exosome markers, were confirmed (Figure 2).

Nanoparticle tracking analysis (NTA) was performed using a particle analyzer (Litesizer 500, Anton Paar) to measure the exosome size. As a result, it was confirmed that the exosomes showed the distribution pattern of general exosomes and that most exosomes were distributed in a size of 50 to 300 nm in diameter (Figure 3).

### <1-4> Isolation of miRNA from exosomes

MiRNAs were extracted from the isolated exosomes using TRIzol reagent (Thermo Fisher Scientific, Waltham, MA, USA).

Specifically, exosomes were lysed by treating with 0.5 ml of TRIzol reagent and incubated at room temperature for 5 minutes, followed by adding 0.1 ml of chloroform and shaking vigorously for 15 seconds to mix. The mixture was incubated at room temperature for 2-3 minutes, centrifuged at 4°C, 12,000 g for 10 minutes, and then the supernatant was transferred to a new tube. RNA was precipitated by adding an equal amount of isopropanol (about 0.25 ml) to the tube and incubating for 10 minutes at room temperature. The RNA pellet was confirmed by centrifugation at 4°C, 12,000 g for 10 minutes, resuspended in 0.5 ml of 75% ethanol, centrifuged at 4°C, 7500 g for 5 minutes, and then the supernatant was removed, and the RNA pellet was dried for 5 minutes. Then, the RNA pellet was dissolved in 0.05 ml of water containing DEPC (Diethyl Pyrocarbonate), resuspended, and placed in a 55°C water bath for 10 minutes.

### <1-5> mRNA sequencing

Library construction was performed using a NEB Next Multiplex Small RNA Library Prep kit (New England BioLabs, Inc., USA) with RNAs from the normal control group and the groups of patients with premature ovarian insufficiency (including both groups of patients with premature ovarian insufficiency caused by Turner syndrome and not caused by Turner syndrome).

Specifically, for the library construction, 1 µg of total RNA of each sample was used to link adapters, and then cDNA was synthesized using reverse transcriptase with adapter-specific primers. PCR was performed to amplify the library, and the library was organized using a QIAquick PCR Purification Kit (Quaigen, Inc, German) and AMPure XP beads (Beckmancoulter, Inc., USA).

The yield and size distribution of the small RNA library were analyzed with the Agilent 2100 Bioanalyzer (Agilent Technologies, Inc., USA) for high sensitivity DNA analysis, and high-throughput sequencing was performed with the NextSeq500 system (Illumina, SanDiego, CA, USA) using single-end 75 sequencing.

### <1-6> Analysis of changes in miRNA expression levels

As a result of miRNA sequencing, 33 miRNAs with varying expression levels were selected (fold change 1.3, normalization 2, significance 0.05) (Table 3).

**[Table 3]**

| miRNA | POI/N | Turner/N |
|---|---|---|
| miR-29a-3p | 0.001 | 0.141 |
| miR-92b-3p | 0.006 | 0.008 |
| miR-16-5p | 0.007 | 0.099 |
| miR-30b-5p | 0.010 | 0.240 |
| miR-18a-5p | 0.017 | 0.015 |
| miR-200a-3p | 0.025 | 0.165 |
| miR-423-3p | 0.028 | 1.803 |
| miR-1260a | 0.046 | 0.162 |
| miR-3184-5p | 0.070 | 3.182 |
| miR-455-5p | 0.074 | 2.640 |
| miR-10a-5p | 0.077 | 0.153 |
| miR-193b-5p | 0.080 | 2.208 |
| miR-125a-5p | 0.105 | 0.109 |
| miR-151a-5p | 0.105 | 0.524 |
| miR-1273a | 0.106 | 0.105 |
| miR-183-5p | 0.110 | 0.136 |
| miR-143-3p | 0.114 | 0.159 |
| let-7c-5p | 0.142 | 0.084 |
| miR-18b-5p | 0.151 | 0.150 |
| miR-92b-5p | 0.185 | 6.274 |
| miR-4452 | 0.199 | 0.203 |
| miR-30d-3p | 0.205 | 0.877 |
| miR-942-5p | 0.251 | 0.227 |
| miR-27b-3p | 0.285 | 0.304 |
| miR-1273g-5p | 0.327 | 1.599 |
| let-7f-5p | 0.348 | 0.554 |
| miR-4508 | 0.361 | 5.372 |
| miR-574-5p | 0.375 | 12.518 |
| miR-26a-5p | 0.403 | 0.245 |
| miR-1273d | 0.679 | 5.582 |
| let-7g-5p | 0.837 | 0.206 |
| miR-125b-5p | 0.854 | 0.289 |
| miR-4516 | 35.549 | 7.254 |

Hierarchical clustering analysis was performed by drawing the distribution of the selected miRNAs as a heat map. As a result, it was confirmed the different miRNA gene expression characteristics between the group of patients with premature ovarian insufficiency and the normal control group (Figure 4). Among them, the expression level of miR-4516 was confirmed to be significantly increased in the group of patients with premature ovarian insufficiency. To confirm the biological action of the target genes of the selected differential expressed genes (DEGs), their distribution was investigated according to gene categories. The results showed that most of the selected miRNAs were involved in uterine embryo development, follicle development, and antral follicle growth, all processes closely related to the development of POI (Figure 5). In the case of other categories, it is believed that they are not included in the category because there is a lack of correlation with premature ovarian insufficiency or because it is a recently discovered mRNA, so no data on the correlation has been secured.

In other words, the changes in the expression levels were observed in the genes involved in uterine embryo development, follicle development, and antral follicle growth, leading to premature ovarian insufficiency due to reduced embryo and follicle development and growth in the uterus.

### Experimental Example 2: Validation and analysis of selected miRNAs

### <2-1> Selection of validation cohort for miRNA validation

Normal people without premature ovarian insufficiency were selected as the control group, and among patients with premature ovarian insufficiency, a group of patients with premature ovarian insufficiency not caused by Turner syndrome and a group of patients with premature ovarian insufficiency caused by Turner syndrome were selected as the experimental groups.

According to the diagnostic method of premature ovarian insufficiency, women with chronic rare ovulation or anovulation with blood folliclestimulating hormone (FSH) of more than 40 mIU/mL measured twice at intervals of more than 1 month were considered as patients with premature ovarian insufficiency, while women with regular menstrual cycles and normal ovulation were selected as the control group.

From June 2020 to July 2021, 15 patients with premature ovarian insufficiency, 11 patients with premature ovarian insufficiency caused by Turner syndrome, and 20 normal people were selected in each group, and analyzed for age, height, weight, BMI, etc. The results are shown in Tables below (Tables 1 and 2).

**[Table 5]**

| | Group of patients with premature ovarian insuffici ency not caused by Turner syndrome (N=16) | Group of patients with premature ovarian insuffici ency caused by Turner syndrome (N=9) | Control (N=20) | P value |
|---|---|---|---|---|
| Age (year) | 35.06±9.9 2 | 34.00±7.3 3 | 34.05±17. 11 | 0.052 |
| Height (cm) | 162.39±6. 38 | 148.91±5. 27 | 163.81±5. 30 | <0.001 |
| Weight (kg) | 59.00±2.4 6 | 55.41±9.4 5 | 51.75±10. 41 | 0.197 |
| BMI (Body mass index) (kg/m²) | 22.48±3.2 7 | 19.57±4.2 7 | 19.24±2.7 2 | 0.340 |
| SBP (systolic blood pressure) (mmHg) | 124.31±4. 35 | 134.11±11 .12 | 122.00±4. 18 | <0.001 |
| DBP (diastolic blood pressure) (mmHg) | 76.06±6.1 7 | 79.89±8.1 0 | 76.70±4.0 4 | 0.217 |
| AST (Aspartate aminotransferase test) (IU/L) | 21.00±5.8 9 | 27.22±14. 32 | 20.85±8.0 3 | 0.224 |
| ALT (Alanine aminotransferase test) (IU/L) | 16.44±7.2 3 | 35.11±35. 59 | 16.50±5.9 2 | 0.022 |
| Total cholesterol (mg/dL) | 185.81±32 .70 | 198.56±20 .38 | 185.70±28 .25 | 0.663 |
| Triglyceride (mg/dL) | 161.56±12 4.18 | 141.80±47 .27 | 110.30±43 .74 | 0.296 |
| HDL-C (highdensity lipoprotein) (mg/dL) | 109.63±41 .12 | 105.04±22 .10 | 98.95±17. 56 | 0.013 |
| LDL-C (lowdensity lipoprotein) (mg/dL) | 109.63±41 .12 | 105.04±22 .10 | 98.95±17. 56 | 0.534 |
| Prolactin (ng/mL) | 8.10±2.86 | 7.76±2.17 | 8.25±2.47 | 0.854 |
| Thyroid stimulating hormone (mIU/mL) | 1.50±1.07 | 2.86±1.00 | 1.70±1.25 | <0.001 |
| Follicular stimulating hormone (mIU/mL) | 92.00±32. 96 | 50.46±26. 74 | 7.2±1.72 | <0.001 |
| Estradiol (pg/mL) | 17.95±12. 58 | 19.72±8.3 7 | 81.27±5.2 7 | <0.001 |

**[Table 6]**

| | Age of menarche (year) | Amenorrh ea period (month) | Karyotype | FMR-1 gene variation |
|---|---|---|---|---|
| Group of patients with premature ovarian insufficiency not caused by Turner syndrome | | | | |
| 1 | No menarche | | 46,XX | No variation |
| 2 | 15 | 18 | 46,XX | No variation |
| 3 | 1516 | 10 | 46,XX | No variation |
| 4 | 1414 | 6 | 46,XX | No variation |
| 5 | 1216 | 70 | 46,XX | No variation |
| 6 | 1215 | 6 | 46,XX,16qh+ | No variation |
| 7 | 11 | 120 | 46,XX | No variation |
| 8 | 13 | 24 | 47,XXX[39]/46,XX[1] | No variation |
| 9 | 14 | 120 | 46,XX,1qh+ | No variation |
| 10 | 15 | 24 | 46,XX | No variation |
| 11 | 13 | 12 | 46,XX | No variation |
| 12 | 14 | 24 | 46,XX | No variation |
| 13 | 12 | 36 | 46,XX | No variation |
| 14 | No menarche | | 46,XX | No variation |
| 15 | 14 | 22 | 46,XX | No variation |

| Group of patients with premature ovarian insufficiency caused by Turner syndrome | | | | |
|---|---|---|---|---|
| 1 | No menarche | | 45,X | No variation |
| 2 | No menarche | | 46,X,i(X)(q10) | No variation |
| 3 | No menarche | | 46,X,i(X)(q10)[24]/45,X[6] | No variation |
| 4 | No menarche | | 45,X | No variation |
| 5 | No menarche | | 45,X[21]/46,X,idic(X)(q25) [9] | No variation |
| 6 | No menarche | | 46,X,i(X)(q10) | No variation |
| 7 | No menarche | | 45,X | No variation |
| 8 | No menarche | | 45,X[17]/46,X,i(X)(q10)[13 ] | No variation |
| 9 | No menarche | | 46,X,i(X)(q10),22pstk+[16] /45,X,22pstk+[14] | No variation |
| 10 | No menarche | | 45,X/46,XX | No variation |
| 11 | No menarche | | 46,X,idic(X)(q22.1)[28]/45 ,X[22] | No variation |

Patients with insufficiency began menarche at approximately 13 or 14 years of age, and patients 1 and 14 did not have menarche as did patients with premature ovarian insufficiency caused by Turner syndrome. These patients had amenorrhea for at least 6 months, and did not have FMR-1 gene mutation, which is analyzed as one of the causes of premature ovarian insufficiency. Among these, patient 8 had karyotypes of 46,XX and 47,XXX, but as it was not considered to be a direct cause of premature ovarian insufficiency, the patient was classified as a patient with premature ovarian insufficiency not caused by Turner syndrome. Patients with premature ovarian insufficiency caused by Turner syndrome showed primary amenorrhea and did not have a mutation in the FMR-1 gene. Some of these patients had a Turner syndrome karyotype with a typical chromosome number abnormality of 45,X, as in patients 1, 4 and 7, while others had a Turner syndrome karyotypes with a chromosomal structural abnormality such as deletions, as in patients 2, 6 and 10. Patients 3, 5, 8, 9 and 11 showed mosaicism of 46,X,i and 45,X, which is a chromosomal structure abnormality.

### <2-2> Isolation of exosomes

Urine was collected from the experimental and control group subjects selected in Experimental Example 2. The collected urine was first centrifuged at 2000 g for 10 minutes to remove cells and sediments, and then centrifuged at 13,500 g for 20 minutes. Afterwards, it was filtered through a 0.22 µm filter and concentrated again using centrifugation (2000 g, 30 minutes).

The exosome-precipitated solution (Exoquick-TC) was mixed at a ratio of 1:4 and incubated at 4°C overnight. The next day, the incubated solution was centrifuged at 2000 g for 30 minutes to isolate the exosome pellet. The isolated pellet was resuspended, combined with beads for 15 minutes, and then centrifuged at 8000 g for 5 minutes to obtain a supernatant containing exosomes.

### <2-3> Isolation of miRNA from exosomes

MiRNAs were extracted from the isolated exosomes using TRIzol reagent (Thermo Fisher Scientific, Waltham, MA, USA).

Specifically, exosomes were lysed by treating with 0.5 ml of TRIzol reagent and incubated at room temperature for 5 minutes, followed by adding 0.1 ml of chloroform and shaking vigorously for 15 seconds to mix. The mixture was incubated at room temperature for 2-3 minutes, centrifuged at 4°C, 12,000 g for 10 minutes, and then the supernatant was transferred to a new tube. RNA was precipitated by adding an equal amount of isopropanol (about 0.25 ml) to the tube and incubating for 10 minutes at room temperature. The RNA pellet was confirmed by centrifugation at 4°C, 12,000 g for 10 minutes, resuspended in 0.5 ml of 75% ethanol, centrifuged at 4°C, 7500 g for 5 minutes, and then the supernatant was removed, and the RNA pellet was dried for 5 minutes.

Then, the RNA pellet was dissolved in 0.05 ml of water containing DEPC, resuspended, and placed in a 55°C water bath for 10 minutes.

### <2-4> miRNA PCR validation and analysis

The relative expressions of the miRNAs isolated in <Experimental Example 2-3> were analyzed using a miRCURY LNA SYBR Green PCR kit (QIAGEN, Hilden, Germany) and miRCURY LNA miRNA PCR assay primers (QIAGEN, Hilden, Germany) for the selected miRNAs.

PCR was performed using CFX384 C1000 thermal cycler (Bio-Rad, Hercules, CA, USA), with an initial thermal activation at 95°C for 2 minutes, followed by 40 cycles of 95°C for 10 seconds and 56°C for 60 seconds, with the PCR cycling conditions of the melting curve analysis.

The sequences of the miRNAs used here and the information on the primers used in the PCR of each miRNA are shown in Table 7.

**[Table 7]**

| microRNA | Primer Cat. no. | miRNA sequence (5'-3') | SEQ. ID. NO: |
|---|---|---|---|
| miR-16-5p | YP00205702 | UAGCAGCACGUAAAUAUUGG CG | 1 |
| miR-20a-5p | YP00204292 | UAAAGUGCUUAUAGUGCAGG UAG | 2 |
| miR-29a-3p | YP00204698 | UAGCACCAUCUGAAAUCGGU UA | 3 |
| miR-30b-5p | YP00204765 | UGUAAACAUCCUACACUCAG CU | 4 |
| miR-99b-3p | YP00204064 | CAAGCUCGUGUCUGUGGGUC CG | 5 |
| miR-151a-5p | YP00204007 | UCGAGGAGCUCACAGUCUAG U | 6 |
| miR-200b-3p | YP00206071 | UAAUACUGCCUGGUAAUGAU GA | 7 |
| miR-423-3p | YP00204488 | AGCUCGGUCUGAGGCCCCUC AGU | 8 |
| miR-941 | YP00204574 | CACCCGGCUGUGUGCACAUG UGC | 9 |
| miR-4516 | YP02112882 | GGGAGAAGGGUCGGGGC | 10 |
| miR-4492 | YP02100455 | GGGGCUGGGCGCGCGCC | 11 |
| miR-7847-3p | YP02105945 | CGUGGAGGACGAGGAGGAGG C | 12 |

From this heat map and significant plot, 12 miRNAs that were either highly differentially expressed in the group of patients with premature ovarian insufficiency not caused by Turner syndrome or in the group of patients with premature ovarian insufficiency caused by Turner syndrome, or that appeared to be evenly expressed in each of the two patient groups were selected (Table 8).

**[Table 8]**

| | |
|---|---|
| Group 1 | A group with high expression differences in the groups of patients with premature ovarian insufficiency |
| | miR-16-5p, miR-29a-3p, miR-30b-5p, miR-151a-5p, miR-423-3p, miR-4516 |
| Group 2 | A group with similar expression levels among the same patient group but with expression differences from the normal group |
| | miR-20a-5p, miR-99b-3p, miR-200b-3p, miR-941, miR-4492, miR-7847-3p |

Comparison of the relative expression levels of the selected miRNAs between the sequencing cohort and the validation cohort is shown in Tables 9 and 10, and Figures 6 and 7. Table 9 shows the expression changes in the miRNA sequencing results of the sequencing cohort and the qRT-PCR verification results of the validation cohort in the group of patients with premature ovarian insufficiency not caused by Turner syndrome and the normal control group. Among the 12 miRNAs selected for validation analysis, 9 miRNAs (miR-16-5p, miR- 29a-3p, miR-30b-5p, miR-99b-3p, miR-151a-5p, miR-423-3p, miR-941, miR-4516 and miR-7847-3p) showed the same expression patterns in the validation cohort of the group of patients with premature ovarian insufficiency not caused by Turner syndrome and in the miRNA sequencing cohort of the group of patients with premature ovarian insufficiency not caused by Turner syndrome (Table 9 and Figure 6).

**[Table 9]**

| miRNA | miRNA sequencing | | | Validation by qRT-PCR | | |
|---|---|---|---|---|---|---|
| | Up/Dow n | Fold change | P value | Up/Dow n | Fold change | P value |
| miR-16-5p | Down | 0.007 | 0.038 | Down | 0.203 | 0.170 |
| miR-20a-5p | Down | 0.009 | 0.101 | Up | 2.410 | 0.361 |
| miR-29a-3p | Down | 0.001 | 0.136 | Down | 0.186 | 0.066 |
| miR-30b-5p | Down | 0.010 | 0.064 | Down | 0.218 | 0.026 |
| miR-99b-3p | Down | 0.060 | 0.254 | Down | 0.505 | 0.185 |
| miR-151a-5p | Down | 0.105 | 0.019 | Down | 0.813 | 0.751 |
| miR-200b-3p | Down | 0.002 | 0.096 | Down | 0.530 | 0.439 |
| miR-423-3p | Down | 0.028 | 0.072 | Down | 0.089 | 0.153 |
| miR-941 | Down | 0.064 | 0.254 | Down | 0.283 | 0.065 |
| miR-4492 | Up | 23.091 | 0.344 | Down | 0.276 | 0.094 |
| miR-4516 | Up | 35.549 | 0.328 | Up | 4.971 | 0.008 |
| miR-7847-3p | Up | 10.175 | 0.085 | Up | 1.449 | 0.568 |

| | | | | | | |
|---|---|---|---|---|---|---|
| *Fold change is expressed as a ratio to the normal control group. In addition, the expression of miR-4516, miR-20a-5p, and miR-200b-3p showed the same expression pattern in the miRNA sequencing cohort and the validation cohort of the group of patients with premature ovarian insufficiency caused by Turner syndrome (Table 10 and Figure 7). | | | | | | |

**[Table 10]**

| | | | | | | |
|---|---|---|---|---|---|---|
| miRNA | miRNA sequencing | | | Validation by qRT-PCR | | |
| | Up/Dow | Fold | *P* | Up/Dow | Fold | *P* |

| | n | change | value | n | change | value |
|---|---|---|---|---|---|---|
| miR-16-5p | Down | 0.099 | 0.058 | Up | 1.673 | 0.811 |
| miR-20a-5p | Up | 1.014 | 0.987 | Up | 1.843 | 0.447 |
| miR-29a-3p | Down | 0.141 | 0.197 | Up | 9.788 | 0.021 |
| miR-30b-5p | Down | 0.240 | 0.284 | Up | 5.380 | 0.119 |
| miR-99b-3p | Up | 14.081 | 0.171 | Down | 0.175 | 0.011 |
| miR-151a-5p | Down | 0.524 | 0.248 | Up | 1.008 | 0.991 |
| miR-200b-3p | Down | 0.692 | 0.671 | Down | 0.439 | 0.296 |
| miR-423-3p | Up | 1.803 | 0.474 | Down | 0.345 | 0.363 |
| miR-941 | Up | 5.138 | 0.235 | Down | 0.207 | 0.064 |
| miR-4492 | Up | 13.250 | 0.058 | Down | 0.286 | 0.107 |
| miR-4516 | Up | 7.254 | 0.044 | Up | 2.663 | 0.022 |
| miR-7847-3p | Up | 12.252 | 0.212 | Down | 0.286 | 0.026 |

| | | | | | | |
|---|---|---|---|---|---|---|
| *Fold change is expressed as a ratio to the normal control group. | | | | | | |

As a result, miR-4516 was expressed significantly higher in both groups of patients with premature ovarian insufficiency not caused by Turner syndrome and premature ovarian insufficiency caused by Turner syndrome compared to the normal control group (Figure 8).

Meanwhile, the expressions of miR-29a-3p and miR-30b-5p were confirmed to be decreased in the group of patients with premature ovarian insufficiency not caused by Turner syndrome, and increased in the group of patients with premature ovarian insufficiency caused by Turner syndrome (Figures 9 and 10).

Since these expressions were different between the groups of patients with premature ovarian insufficiency and patients with Turner syndrome, they can be used as biomarkers to distinguish between patients with premature ovarian insufficiency and patients with Turner syndrome.

Therefore, if miR-4516 is increased and miR-29a-3p and miR-30b-5p are decreased, the patient can be judged to have premature ovarian insufficiency not caused by Turner syndrome, and if miR-4516 is increased and miR- 29a-3p and miR-30b-5p are also increased, the patient can be judged to have premature ovarian insufficiency caused by Turner syndrome.

### Experimental Example 3: Confirmation of increased expression of miR-4516 in premature ovarian insufficiency mouse model

### <3-1> Establishment of premature ovarian insufficiency mouse model for miRNA confirmation

Eight-week-old female Specific pathogen free (SPF) grade C57B6L/N mice were obtained from Orient Bio Inc. (Seongnam, Korea). Mice were maintained at controlled temperature (20-22°C) and light (12/12 hr light/dark cycle) and had free access to water and food. The animals were randomly divided into two groups: control (n=7) and CTX+BUS-POI (n=7), and three independent experiments were performed. To induce premature ovarian insufficiency, mice were injected intraperitoneally with 120 mg/kg of CTX (cyclophosphamide) and 12 mg/kg of BUS (busulfan) three times a week for 2 weeks, and saline was injected as a control group. All the experimental procedures and maintenance and care in this study were conducted in accordance with the Animal Care and Use Center, Gachon University Lee Gil-Yeo Cancer and Diabetes Research Institute, and the Institutional Animal Care and Use Committee (IACUC) (Accession Number: LCDI-2021-0121). CTX (C0768) and BUS (B2635) were purchased from Sigma-Aldrich (St. Louis, MO, USA).

### <3-2> Hematoxylin and eosin (H&E) staining and Sirius red staining of ovaries of premature ovarian insufficiency mouse model

Ovarian tissues were fixed in 4% paraformaldehyde overnight, dehydrated, embedded in paraffin, and cut into 5 µm thick sections. The fifth section was dewaxed, rehydrated, and stained with hematoxylin and eosin (H&E) or Sirius red for microscopic observation. H&E staining stains the nucleus with hematoxylin and the cytoplasm with eosin. Sirius red staining was performed to confirm collagen fibers in the ovarian tissue. H&E staining and Sirius red staining images were randomly captured using a Motic Easyscan Digital Slide Scanner (Motic Hong Kong Limited, Hong Kong, China) .

### <3-3> TUNEL assay and Ki67 immunohistochemistry of ovaries of premature ovarian insufficiency mouse model

Apoptosis in the ovarian tissue was confirmed using DeadEnd^{™} Fluorometric TUNEL System (G3250, Promega, Fitchburg, WI, USA) according to the manufacturer's instructions. After preparing the mounting medium for fluorescence with DAPI (4',6-diamidino-2-phenylindole,Vector Lab), the slides were examined using Olympus CKX3-Houn microscope (Olympus, Tokyo, Japan). To complete Ki67 immunohistochemistry, the sections were incubated in 0.01 M citrate buffer (pH 6.0), deparaffinized and rehydrated, and then antigen retrieval was performed by applying intense microwave irradiation for 30 minutes. Endogenous peroxidase activity was inhibited with 3% H₂O₂ for 10 minutes, and non-specific binding was blocked with 10% normal goat serum for 1 hour. The sections were treated with Ki-67 primary antibody (1:200; ab16667Abcam, UK) for 24 hours at 4°C. Antigens were visualized using diaminobenzidine (DAB). Finally, the slides were dehydrated, counterstained with hematoxylin, and mounted. Ki67 immunohistochemistry images were captured randomly using a Motic Easyscan digital slide scanner (Motic Hong Kong Limited, Hong Kong, China).

### <3-4> Isolation of miRNAs from mouse ovary and uterus

Total RNA was extracted from the ovary and uterus using TRIzol reagent (15596026, Thermo Fisher Scientific) according to the manufacturer's instructions. Specifically, the tissues were pulverized and treated with 0.5 ml TRIzol reagent to lysis and incubated at room temperature for 5 min, followed by the addition of 0.1 ml chloroform and vigorous shaking for 15 seconds to mix. The mixture was incubated at room temperature for 2-3 minutes, centrifuged at 4°C, 12,000 g for 10 minutes, and the supernatant was transferred to a new tube. RNA was precipitated by adding an equal amount of isopropanol (about 0.25 ml) and incubating for 10 minutes at room temperature. The mixture was centrifuged at 4°C, 12,000g for 10 minutes to confirm RNA pellet, resuspended in 0.5 ml of 75% ethanol, and centrifuged at 4°C, 7500g for 5 minutes. The supernatant was removed, and the RNA pellet was dried for 5 minutes. Then, the RNA pellet was dissolved in 0.05 ml of water containing DEPC, resuspended, and incubated in a 55°C water bath for 10 minutes.

### <3-5> miRNA PCR validation and analysis

The relative expressions of the miRNAs isolated in <Experimental Example 3-4> were analyzed using a miRCURY LNA SYBR Green PCR kit (QIAGEN, Hilden, Germany) and miRCURY LNA miRNA PCR assay primers (QIAGEN, Hilden, Germany) for the selected miRNAs. The miRCURY LNA RT kit (339340, QIAGEN, Maryland, Germany) was used to synthesize cDNA from 5 ng of total RNA using 10x miRCURY RT Enzyme Mix containing UniSp6 RNA spike-in (QIAGEN, Germany) in a reaction volume of 20 µL. Quantitative real-time polymerase chain reaction (qRT-PCR) was performed using CFX384 C1000 Thermal Cycler (Bio-Rad, Hercules, CA, USA) with 2x miRCURY SYBER Green Master Mix (QIAGEN). The expression level was analyzed as the relative expression to UniSp6 (Kinoshita et al., 2017). The miRCURY LNA miRNA SYBR Green PCR primers used in this study are shown in Table 7.

PCR was performed using CFX384 C1000 thermal cycler (Bio-Rad, Hercules, CA, USA), with an initial thermal activation at 95°C for 2 minutes, followed by 40 cycles of 95°C for 10 seconds and 56°C for 60 seconds, with the PCR cycling conditions of the melting curve analysis.

### <3-6> Expression analysis of proteins in premature ovarian insufficiency mouse model

Proteins in the mouse ovary were extracted using T-PER Tissue Protein Extraction Reagent supplemented with a protease (Roche, Mannheim, Germany) and a phosphatase inhibitor (Roche). Ovarian tissue (30 mg) was homogenized in an extraction buffer using TissueLyser II (Qiagen, Hilden, Germany) at 30 Hz for 1-2 minutes. The lysate was centrifuged at 13,000 x g for 10 minutes at 4°C to remove debris. The supernatant was mixed with a sodium dodecyl sulfate sample buffer and boiled for 5 minutes. The proteins were electrophoresed on an SDS-polyacrylamide gel and then transferred to a polyvinylidene fluoride membrane (Millipore, Billerica, MA, USA). The membrane was incubated with primary antibodies according to the manufacturer's instructions. HRP(horseradish peroxidase)-conjugated secondary antibodies were detected using an Immobilon Western Chemiluminescent HRP substrate (Millipore). The band intensities of Western blots were quantified by densitometry using ImageJ software.

To evaluate miR-4516 as a premature ovarian insufficiency biomarker, the present inventors established a mouse model of chemotherapy-induced premature ovarian insufficiency by injecting mice with CTX and BUS. In the premature ovarian insufficiency mouse model, there was no apparent death or toxicity, but the size of the ovaries was smaller than in the saline-injected control group mice (Figures 11a and 11b). Compared to the saline-injected control group mice, the premature ovarian insufficiency group mice had a significant decrease in follicle number and an increase in the ratio of atretic follicles/total follicles (Figures 12a to 12c). There was also an increase in Sirius red staining, which indicates collagen deposition, suggesting that tissue fibrosis is progressing (Figure 13). In addition, TUNEL-positive cells were significantly increased in the premature ovarian insufficiency group (Figures 14a and 14b), while Ki67-positive cells were decreased (Figure 15). These results confirmed that the premature ovarian insufficiency mouse model was successfully established by chemotherapy. Next, the present inventors confirmed the expression of miR-4516 in the ovaries of the chemotherapy-induced premature ovarian insufficiency mouse model. As shown in Figures 16a and 16b, the expression of miR-4516 was significantly increased in the ovaries of the premature ovarian insufficiency mouse model compared to the control group, but not in the uterus. These results indicate that the increased expression of miR-4516 is associated with the pathological changes in the ovary.

To interpret the pathological relevance of the miR-4516 overexpression, the present inventors analyzed the targets of miR-4516 using miRTarBase2020, an experimentally validated miRNA-target interaction database (Huang et al., 2020). By this analysis method, STAT3 was confirmed as a target of miR-4516 with experimental evidence (Figure 17). The 3'-UTR region of STAT3 contains two putative miR-4516 binding sites (Chowdhari and Saini, 2014) (Figure 17). Consistent with this interaction prediction, STAT3 protein expression was significantly reduced in the ovaries of the chemotherapy-induced premature ovarian insufficiency mouse model, along with increased expression of the apoptosis markers Bax and p53 (Figures 18a to 18d). These results suggest that increasing miR-4516 is closely associated with POI pathology along with decreasing STAT3 expression. It is believed that the regulation of STAT3 expression by miR-4516 is involved in cell death, egg maturation, and primodal cell activation (Figure 19).

## Claims

1. An *in vitro* method for diagnosing premature ovarian insufficiency (POI), comprising the step of measuring the expression level of miRNAs consisting of MiR-4516, MiR-29a-3p, and MiR-30b-5p, and
wherein
(a) when the expression level of miR-4516 is increased compared to the normal control group and the expression level of miR-29a-3p is decreased compared to the normal control group, the subject is determined to be at high risk of premature ovarian insufficiency not caused by Turner syndrome,
(b) when the expression level of miR-4516 is increased compared to the normal control group and the expression level of miR-30b-5p is decreased compared to the normal control group, the subject is determined to be at high risk of premature ovarian insufficiency not caused by Turner syndrome,
(c) when the expression level of miR-4516 is increased compared to the normal control group and the expression level of miR-29a-3p and miR-30b-5p is increased compared to the normal control group, the subject is determined to be at high risk of premature ovarian insufficiency caused by Turner syndrome,
(d) when the expression level of miR-4516 is increased compared to the normal control group and the expression level of miR-29a-3p and miR-30b-5p is decreased compared to the normal control group, the subject is determined to be at high risk of premature ovarian insufficiency not caused by Turner syndrome,
(e) when the expression level of miR-4516 and miR-29a-3p is increased compared to the normal control group, the subject is determined to be at high risk of premature ovarian insufficiency caused by Turner syndrome, and
(f) when the expression level of miR-4516 and miR-30b-5p is increased compared to the normal control group, the subject is determined to be at high risk of premature ovarian insufficiency caused by Turner syndrome.

2. The *in vitro* method according to claim 1, wherein the miRNA is RNA obtained from exosomes isolated from a biological sample, and
wherein the biological sample is cell culture supernatant, whole blood, serum, plasma, ascitic fluid, cerebrospinal fluid, bone marrow aspirate, bronchoalveolar lavage, urine, vaginal fluid, mucus, saliva, sputum, or a lysate purified from a biosample.

3. An *in vitro* method for screening a therapeutic agent for premature ovarian insufficiency comprising the following steps:
1) a step of treating a test substance to cells having a nucleotide sequence encoding miRNAs consisting of miR-4516, miR-29a-3p, and miR-30b-5p in vitro,
2) a step of measuring the expression level of miRNA in the isolated cells treated with the test substance and comparing the expression pattern of the miRNA with the expression level of the miRNA in the isolated control cells not treated with the test substance; and
wherein
(a) when the expression level of miR-4516 is increased compared to the normal control group and the expression level of miR-29a-3p is decreased compared to the normal control group, the subject is determined to be at high risk of premature ovarian insufficiency not caused by Turner syndrome,
(b) when the expression level of miR-4516 is increased compared to the normal control group and the expression level of miR-30b-5p is decreased compared to the normal control group, the subject is determined to be at high risk of premature ovarian insufficiency not caused by Turner syndrome,
(c) when the expression level of miR-4516 is increased compared to the normal control group and the expression level of miR-29a-3p and miR-30b-5p is increased compared to the normal control group, the subject is determined to be at high risk of premature ovarian insufficiency caused by Turner syndrome,
(d) when the expression level of miR-4516 is increased compared to the normal control group and the expression level of miR-29a-3p and miR-30b-5p is decreased compared to the normal control group, the subject is determined to be at high risk of premature ovarian insufficiency not caused by Turner syndrome,
(e) when the expression level of miR-4516 and miR-29a-3p is increased compared to the normal control group, the subject is determined to be at high risk of premature ovarian insufficiency caused by Turner syndrome, and
(f) when the expression level of miR-4516 and miR-30b-5p is increased compared to the normal control group, the subject is determined to be at high risk of premature ovarian insufficiency caused by Turner syndrome.

4. The in vitro method according to claim 3, wherein the agent is a nucleic acid containing a polynucleotide identical to or complementary to the nucleic acid sequence of the miRNA.

5. The in vitro method according to claim 4, wherein the nucleic acid is a primer or probe.

6. The in vitro method according to claim 3, wherein the miRNA is RNA obtained from exosomes isolated from a biological sample, and
wherein the biological sample is cell culture supernatant, whole blood, serum, plasma, ascitic fluid, cerebrospinal fluid, bone marrow aspirate, bronchoalveolar lavage, urine, vaginal fluid, mucus, saliva, sputum, or a lysate purified from a biosample.

## Patentansprüche

1. In-vitro-Verfahren zur Diagnose einer prämaturen Ovarialinsuffizienz (POI), umfassend den Schritt des Messens des Expressionsniveaus von miRNAs, bestehend aus miR-4516, miR-29a-3p und miR-30b-5p, und
wobei
(a) wenn das Expressionsniveau von miR-4516 im Vergleich zur normalen Kontrollgruppe erhöht ist und das Expressionsniveau von miR-29a-3p im Vergleich zur normalen Kontrollgruppe verringert ist, bei der Probandin ein hohes Risiko für eine prämature Ovarialinsuffizienz festgestellt wird, die nicht durch das Turner-Syndrom verursacht wird,
(b) wenn das Expressionsniveau von miR-4516 im Vergleich zur normalen Kontrollgruppe erhöht ist und das Expressionsniveau von miR-30b-5p im Vergleich zur normalen Kontrollgruppe verringert ist, bei der Probandin ein hohes Risiko für eine prämature Ovarialinsuffizienz festgestellt wird, die nicht durch das Turner-Syndrom verursacht wird,
(c) wenn das Expressionsniveau von miR-4516 im Vergleich zur normalen Kontrollgruppe erhöht ist und das Expressionsniveau von miR-29a-3p und miR-30b-5p im Vergleich zur normalen Kontrollgruppe erhöht ist, bei der Probandin ein hohes Risiko für eine prämature Ovarialinsuffizienz festgestellt wird, die durch das Turner-Syndrom verursacht wird,
(d) wenn das Expressionsniveau von miR-4516 im Vergleich zur normalen Kontrollgruppe erhöht ist und das Expressionsniveau von miR-29a-3p und miR-30b-5p im Vergleich zur normalen Kontrollgruppe verringert ist, bei der Probandin ein hohes Risiko für eine prämature Ovarialinsuffizienz festgestellt wird, die nicht durch das Turner-Syndrom verursacht wird,
(e) wenn das Expressionsniveau von miR-4516 und miR-29a-3p im Vergleich zur normalen Kontrollgruppe erhöht ist, bei der Probandin ein hohes Risiko für eine prämature Ovarialinsuffizienz festgestellt wird, die durch das Turner-Syndrom verursacht wird, und
(f) wenn das Expressionsniveau von miR-4516 und miR-30b-5p im Vergleich zur normalen Kontrollgruppe erhöht ist, bei der Probandin ein hohes Risiko für eine prämature Ovarialinsuffizienz festgestellt wird, die durch das Turner-Syndrom verursacht wird.

2. In-vitro-Verfahren gemäß Anspruch 1, wobei die miRNA eine RNA ist, die aus Exosomen gewonnen wird, die aus einer biologischen Probe isoliert wurden, und
wobei die biologische Probe Zellkulturüberstand, Vollblut, Serum, Plasma, Aszitesflüssigkeit, Liquor cerebrospinalis, Knochenmarkaspirat, bronchoalveoläre Lavage, Urin, Vaginalflüssigkeit, Schleim, Speichel, Sputum oder ein aus einer biologischen Probe gereinigtes Lysat ist.

3. In-vitro-Verfahren zum Screenen eines therapeutischen Wirkstoffs für prämature Ovarialinsuffizienz, umfassend die folgenden Schritte:
1) einen Schritt, bei dem Zellen, die eine Nukleotidsequenz aufweisen, die miRNAs bestehend aus miR-4516, miR-29a-3p und miR-30b-5p, kodieren, mit einer Testsubstanz in vitro behandelt werden,
2) einen Schritt des Messens des Expressionsniveaus von miRNA in den isolierten Zellen, die mit der Testsubstanz behandelt wurden, und des Vergleichens des Expressionsmusters der miRNA mit dem Expressionsniveau der miRNA in den isolierten Kontrollzellen, die nicht mit der Testsubstanz behandelt wurden; und
wobei
(a) wenn das Expressionsniveau von miR-4516 im Vergleich zur normalen Kontrollgruppe erhöht ist und das Expressionsniveau von miR-29a-3p im Vergleich zur normalen Kontrollgruppe verringert ist, bei der Probandin ein hohes Risiko für eine prämature Ovarialinsuffizienz festgestellt wird, die nicht durch das Turner-Syndrom verursacht wird,
(b) wenn das Expressionsniveau von miR-4516 im Vergleich zur normalen Kontrollgruppe erhöht ist und das Expressionsniveau von miR-30b-5p im Vergleich zur normalen Kontrollgruppe verringert ist, bei der Probandin ein hohes Risiko für eine prämature Ovarialinsuffizienz festgestellt wird, die nicht durch das Turner-Syndrom verursacht wird,
(c) wenn das Expressionsniveau von miR-4516 im Vergleich zur normalen Kontrollgruppe erhöht ist und das Expressionsniveau von miR-29a-3p und miR-30b-5p im Vergleich zur normalen Kontrollgruppe erhöht ist, bei der Probandin ein hohes Risiko für eine prämature Ovarialinsuffizienz festgestellt wird, die durch das Turner-Syndrom verursacht wird,
(d) wenn das Expressionsniveau von miR-4516 im Vergleich zur normalen Kontrollgruppe erhöht ist und das Expressionsniveau von miR-29a-3p und miR-30b-5p im Vergleich zur normalen Kontrollgruppe verringert ist, bei der Probandin ein hohes Risiko für eine prämature Ovarialinsuffizienz festgestellt wird, die nicht durch das Turner-Syndrom verursacht wird,
(e) wenn das Expressionsniveau von miR-4516 und miR-29a-3p im Vergleich zur normalen Kontrollgruppe erhöht ist, bei der Probandin ein hohes Risiko für eine prämature Ovarialinsuffizienz festgestellt wird, die durch das Turner-Syndrom verursacht wird, und
(f) wenn das Expressionsniveau von miR-4516 und miR-30b-5p im Vergleich zur normalen Kontrollgruppe erhöht ist, bei der Probandin ein hohes Risiko für eine prämature Ovarialinsuffizienz festgestellt wird, die durch das Turner-Syndrom verursacht wird.

4. In-vitro-Verfahren gemäß Anspruch 3, wobei das Mittel eine Nukleinsäure ist, die ein Polynukleotid enthält, das identisch mit oder komplementär zu der Nukleinsäuresequenz der miRNA ist.

5. In-vitro-Verfahren gemäß Anspruch 4, wobei die Nukleinsäure ein Primer oder eine Sonde ist.

6. In-vitro-Verfahren nach Anspruch 3, wobei die miRNA eine RNA ist, die aus Exosomen gewonnen wird, die aus einer biologischen Probe isoliert wurden, und
wobei die biologische Probe Zellkulturüberstand, Vollblut, Serum, Plasma, Aszitesflüssigkeit, Liquor cerebrospinalis, Knochenmarkaspirat, bronchoalveoläre Lavage, Urin, Vaginalflüssigkeit, Schleim, Speichel, Sputum oder ein aus einer biologischen Probe gereinigtes Lysat ist.

## Revendications

1. Procédé *in vitro* pour diagnostiquer l'insuffisance ovarienne prématurée (IOP), comprenant l'étape de mesure du niveau d'expression des miARN constitués par miR-4516, miR-29a-3p et miR-30b-5p, et
dans lequel
(a) lorsque le niveau d'expression de miR-4516 est augmenté par rapport au groupe témoin normal et que le niveau d'expression de miR-29a-3p est réduit par rapport au groupe témoin normal, il est déterminé que le sujet est à haut risque d'insuffisance ovarienne prématurée non causée par le syndrome de Turner,
(b) lorsque le niveau d'expression de miR-4516 est augmenté par rapport au groupe témoin normal et que le niveau d'expression de miR-30b-5p est réduit par rapport au groupe témoin normal, il est déterminé que le sujet est à haut risque d'insuffisance ovarienne prématurée non causée par le syndrome de Turner,
(c) lorsque le niveau d'expression de miR-4516 est augmenté par rapport au groupe témoin normal et que le niveau d'expression de miR-29a-3p et miR-30b-5p est augmenté par rapport au groupe témoin normal, il est déterminé que le sujet est à haut risque d'insuffisance ovarienne prématurée causée par le syndrome de Turner,
(d) lorsque le niveau d'expression de miR-4516 est augmenté par rapport au groupe témoin normal et que le niveau d'expression de miR-29a-3p et miR-30b-5p est réduit par rapport au groupe témoin normal, il est déterminé que le sujet est à haut risque d'insuffisance ovarienne prématurée non causée par le syndrome de Turner,
(e) lorsque le niveau d'expression de miR-4516 et miR-29a-3p est augmenté par rapport au groupe témoin normal, il est déterminé que le sujet est à haut risque d'insuffisance ovarienne prématurée causée par le syndrome de Turner, et
(f) lorsque le niveau d'expression de miR-4516 et miR-30b-5p est augmenté par rapport au groupe témoin normal, il est déterminé que le sujet est à haut risque d'insuffisance ovarienne prématurée causée par le syndrome de Turner.

2. Procédé *in vitro* selon la revendication 1, dans lequel le miARN est de l'ARN obtenu à partir d'exosomes isolés d'un échantillon biologique, et
dans lequel l'échantillon biologique est du surnageant de culture cellulaire, du sang total, du sérum, du plasma, du liquide ascitique, du liquide céphalorachidien, du liquide de ponction de moelle osseuse, du liquide de lavage broncho-alvéolaire, de l'urine, des sécrétions vaginales, du mucus, de la salive, du crachat ou un lysat purifié provenant d'un échantillon biologique.

3. Procédé *in vitro* pour détecter un agent thérapeutique pour l'insuffisance ovarienne prématurée comprenant les étapes suivantes :
1) une étape de traitement avec une substance d'essai des cellules ayant une séquence nucléotidique codant pour des miARN constitués par miR-4516, miR-29a-3p et miR-30b-5p, *in vitro,*
2) une étape de mesure du niveau d'expression du miARN dans les cellules isolées traitées avec la substance d'essai et de comparaison du profil d'expression du miARN avec le niveau d'expression du miARN dans les cellules témoins isolées non traitées avec la substance d'essai ; et
dans lequel
(a) lorsque le niveau d'expression de miR-4516 est augmenté par rapport au groupe témoin normal et que le niveau d'expression de miR-29a-3p est réduit par rapport au groupe témoin normal, il est déterminé que le sujet est à haut risque d'insuffisance ovarienne prématurée non causée par le syndrome de Turner,
(b) lorsque le niveau d'expression de miR-4516 est augmenté par rapport au groupe témoin normal et que le niveau d'expression de miR-30b-5p est réduit par rapport au groupe témoin normal, il est déterminé que le sujet est à haut risque d'insuffisance ovarienne prématurée non causée par le syndrome de Turner,
(c) lorsque le niveau d'expression de miR-4516 est augmenté par rapport au groupe témoin normal et que le niveau d'expression de miR-29a-3p et miR-30b-5p est augmenté par rapport au groupe témoin normal, il est déterminé que le sujet est à haut risque d'insuffisance ovarienne prématurée causée par le syndrome de Turner,
(d) lorsque le niveau d'expression de miR-4516 est augmenté par rapport au groupe témoin normal et que le niveau d'expression de miR-29a-3p et miR-30b-5p est réduit par rapport au groupe témoin normal, il est déterminé que le sujet est à haut risque d'insuffisance ovarienne prématurée non causée par le syndrome de Turner,
(e) lorsque le niveau d'expression de miR-4516 et miR-29a-3p est augmenté par rapport au groupe témoin normal, il est déterminé que le sujet est à haut risque d'insuffisance ovarienne prématurée causée par le syndrome de Turner, et
(f) lorsque le niveau d'expression de miR-4516 et miR-30b-5p est augmenté par rapport au groupe témoin normal, il est déterminé que le sujet est à haut risque d'insuffisance ovarienne prématurée causée par le syndrome de Turner.

4. Procédé *in vitro* selon la revendication 3, dans lequel l'agent est un acide nucléique contenant un polynucléotide identique ou complémentaire à la séquence d'acides nucléiques du miARN.

5. Procédé *in vitro* selon la revendication 4, dans lequel l'acide nucléique est une amorce ou une sonde.

6. Procédé *in vitro* selon la revendication 3, dans lequel le miARN est de l'ARN obtenu à partir d'exosomes isolés d'un échantillon biologique, et
dans lequel l'échantillon biologique est du surnageant de culture cellulaire, du sang total, du sérum, du plasma, du liquide ascitique, du liquide céphalorachidien, du liquide de ponction de moelle osseuse, du liquide de lavage broncho-alvéolaire, de l'urine, des sécrétions vaginales, du mucus, de la salive, du crachat ou un lysat purifié provenant d'un échantillon biologique.
